# EUROPEAN PATENT APPLICATION

(11) **EP 4 465 048 A1**
(43) Date of publication of application: **20.11.2024**
(21) Application number: 23173570.5
(22) Date of filing: 16.05.2023
(51) Int. Cl.: G01N 33/574, G01N 33/72

(54) **QUICK TEST COMBINATION FOR SCREENING OF DYSPEPSIA AND OTHER ABDOMINAL SYMPTOMS**

(71) Applicant: BIOHIT OYJ, 00880 Helsinki (FI)
(72) Inventor: SUOVANIEMI, Osmo, 00570 Helsinki (FI)
(74) Representative: Laine IP Oy

(57) **Abstract**

According to an example aspect of the present invention, there is provided a novel combination of two non-invasive tests and their application in the first-line detection of subjects with dyspepsia and other non-specific abdominal symptoms.

## Description

### FIELD

The present invention relates to a method for screening of a symptomless subject or a subject having dyspeptic, reflux or other abdominal symptoms, by carrying out two parallel non-invasive quantitative biomarker measurements from samples obtained from said subject.

### BACKGROUND

Dyspepsia belongs among the most frequent clinical symptoms experienced by 20-40% of the people during their lifetime. Symptoms of dyspepsia are non-specific and cannot be distinguished from a number of clinically important diseases of extra-gastric origin, including colorectal neoplasia. Far too often, the current clinical practice offers invasive endoscopy as the first-line diagnostic test for these patients. Alternative diagnostic algorithms suggest a rational use of non-invasive tests targeting the specific gastrointestinal disorders.

Both GastroPanel^{®} ELISA and GastroPanel^{®} Quick test is a combination of four stomach-specific biomarkers (pepsinogen I, pepsinogen II, gastrin-17 and *Helicobacter pylori* IgG antibody ELISA) designed for the first-line diagnosis of patients with symptomatic dyspepsia, as well as for screening of asymptomatic subjects for gastric cancer (GC) risk conditions: atrophic gastritis (AG) and *Helicobacter pylori* (Hp) infection. In addition, GastroPanel^{®} test also accurately monitors the status of gastric acid output by measuring the PGI-G-17 balance.

The latest version of GastroPanel^{®} ELISA is known as unified GastroPanel. In this assay, performed in the laboratory, all four markers are being processed under the same conditions using an automatic ELISA instrument or manual processing. In turn, GastroPanel^{®} Quick test can be performed during a single clinical appointment, saving costs and unnecessary clinical appointments and speeding up the referral to further examinations. It is a lateral-flow test based on immunoassay method detecting the four biomarkers from blood (whole blood, plasma, or serum) samples obtained from the fingertip blood drop. Dispensed samples are transferred with transposable transfer pipettes from the sample tubes into the four sample holes (for each biomarker; 100 µl into each hole) of the lateral-flow test cassette. The cassette is then measured after 15 minutes with the GastroPanel Reader instrument. Results can be read from the reader screen and printed. It shows both numerical values for each biomarker as well as the interpretation based on a similar diagnostic algorithm as the original ELISA version.

Both ColonView^{®} Quick test and ColonView^{®} ELISA represent second generation fecal immunochemical tests (FIT) designed for detection of fecal occult blood (FOB) in a point-of-care diagnostic setting and for screening of CRC. Compared with other FIT tests, ColonView^{®} Quick test has the unique feature of using two biomarkers for FOB detection: Hb and Hb/Hp complex. Testing for both FOB markers increases test sensitivity, particularly for the neoplasia localized in the proximal colon.

Dyspeptic symptoms are among the most common abdominal complaints [1], experienced by 25-40% of the people during their lifetime [1, 2]. Within the primary health care, most of these patients are primarily treated (e.g. with proton pump inhibitors, PPI) without confirmation of the proper diagnosis at first [3-5]. Majority of these complaints are due to functional dyspepsia or gastro-esophageal reflux disease (GERD), while a small minority are classified organic in origin [4, 6]. Of the latter, the two most important clinical conditions are *Helicobacter pylori* (Hp) infection and atrophic gastritis (AG), two conditions that are closely interrelated [7, 8].

Hp is the causal agent for clinically important diseases in gastric and duodenal mucosa [7, 9-11], and, in 1994, IARC classified HP-infection as group I human carcinogen [12]. This bacterial infection initially affects only the antral mucosa causing superficial gastritis. If not eradicated, Hp-infection progresses to chronic corpus-predominant gastritis or pangastritis, with mucosal atrophy as the end result [13-25].

In addition to the conditions of gastric origin, also other well-defined clinical diseases cause non-specific (multiple type) clinical symptoms sharing many features in common with dyspepsia. Such conditions include e.g. celiac disease (CD) [26-28], lactose intolerance (LI) [29-32], inflammatory bowel disease (IBD) [33-36], irritable bowel syndrome (IBS) [33-36] and neoplastic lesions of the colon [37]. The clinical symptoms in all these conditions are non-specific enough to preclude the correct diagnosis on clinical basis alone [26-37].

Particularly among elderly people, non-specific abdominal symptoms can be of intestinal origin, possibly caused by colorectal cancer (CRC). CRC is among the major global cancers with increasing disease burden in most of the western countries. At present, several countries have implemented or currently plan to implement, organized population-based CRC screening programs. CRC screening can impact both i) the primary prevention (finding precursor lesions with malignant potential) and ii) the secondary prevention (detecting early-stage cancers amenable to treatment) [38-40].

In the lack of an ideal test for CRC screening, guaiac-based fecal occult blood test (gFOBT) and fecal immunochemical test (FIT) have been used for CRC screening in countries where population-based programs are implemented [40]. gFOBTs are no longer the preferred test for newly designed CRC screening programs, but quantitative FITs are currently the test of choice for population-based screening and in diagnosis of FOB in non-screening settings [38, 39].

Several diagnostic tests are available for dyspeptic symptoms, including endoscopy, radiography and testing for Hp-infection [25]. In patients with the symptoms suspected to be of stomach origin, endoscopy with targeted biopsies remains the gold standard diagnostic tool, disclosing Hp-infection, AG, IN as well as their topography [8, 14]. However, this invasive method is uncomfortable, distressing and quite costly, which precludes its use as the diagnostic tool in population-based screening. This emphasizes the need for rapid, accurate and inexpensive non-invasive tests for screening and monitoring of the patients with dyspeptic symptoms [23-25].

### SUMMARY OF THE INVENTION

The invention is defined by the features of the independent claims. Some specific embodiments are defined in the dependent claims.

This document describes a novel combination of two test versions (ELISA or Quick Test) of two non-invasive tests: GastroPanel^{®} and ColonView^{®} (Biohit Oyj, Helsinki) and their application in the first-line detection of subjects with dyspepsia and other non-specific abdominal symptoms.

Thus, according to one aspect of the present invention, there is provided a method for non-invasive screening of a symptomless subject or subject having reflux, dyspeptic or other abdominal symptoms.

This and other aspects, together with the advantages thereof over known solutions are achieved by the present invention, as hereinafter described and claimed.

The method of the present invention is mainly characterized by what is stated in the characterizing part of claim 1.

Considerable advantages are obtained by means of the invention. The combined use of GastroPanel^{®} and ColonView^{®} tests (any combination of the two test versions) offers several advantages over invasive diagnostic methods (gastroscopy and colonoscopy). GastroPanel^{®} (ELISA or Quick Test version) as the first-line test offers a possible to stratify the patients into three categories at different risk for GC. Low Risk: Because of the very high negative predictive value (NPV) of GastroPanel^{®} test, the patients with normal biomarker profile should be examined for disorders of extra-gastric origin, e.g. by ColonView^{®} test. The intermediate risk category includes the subjects testing Hp-positive with no indication of AG. For those patients, Hp-eradication should be done, followed by appropriate control. The patients at high risk for GC include all those with AG, who need a referral to gastroscopy to confirm the AG, its severity and topography in the stomach. Whenever FOB testing is indicated, ColonView^{®} Quick test or Colon View ELISA offers the best sensitivity when three consecutive fecal samples are tested for both Hb and Hb/Hp complex. This protocol far exceeds the sensitivity achieved by i) the FITs using Hb testing only, or by ii) any test protocol analyzing one fecal sample only.

Taken together, replacing gastroscopy by GastroPanel^{®} (ELISA or Quick test) and colonoscopy by ColonView^{®} (ELISA or Quick test) as the first-line tests in patients with dyspepsia and other non-specific abdominal symptoms allows a rational algorithm whereby these non-invasive tests are applied to detect gastric and extra-gastric conditions sharing in common the non-specific clinical symptoms. Substantial savings in health care costs can be achieved while avoiding up to 80% of unnecessary gastroscopies and majority of colonoscopies by examining these patients first by GastroPanel^{®} test, followed by ColonView^{®} Quick test, whenever an extra-gastric disorder is suspected.

Next, the present technology will be described more closely with reference to certain embodiments.

### EMBODIMENTS

In the public health sector, cost savings are the global issue, dictated by the restricted public health investments in most countries. As an example, Finnish standard care for the primary diagnosis of dyspepsia does not recommend biomarkers or fecal blood determination, which would enable early diagnosis to avoid unnecessary costs and deaths. Another "mega-trend" is a tendency to replace invasive diagnostic tests by non-invasive tests as far as possible. A clear advantage of many of these non-invasive tools is their versatility and adaptability for immediate testing at the outpatient department or at doctor's office, i.e. point-of-care (POC) testing.

The present technology provides a rational combined use of either test versions of GastroPanel^{®} and ColonView^{®} tests (ELISA or Quick test version) in the algorithm for the patients who search medical attention due to dyspepsia or non-specific abdominal symptoms.

The proposed algorithm is based on the fact that invasive gastroscopy [8, 14, 23-25] is replaced by non-invasive GastroPanel^{®} test as the first-line tool [43-51]. GastroPanel^{®} test is a biomarker panel of four stomach-specific biomarkers (PGI, PGII, G-17, Hp IgG Ab ELISA) [43-51]. The results are interpreted by a special software (GastroSoft^{®}) that distinguishes eight distinct marker profiles [42, 43, 46, 51]. These refined categories can be stratified to three groups with different risk and different management indications: LOW RISK: normal marker profile; INTERMEDIATE RISK: Hp-infection (with no atrophy), and HIGH RISK: Atrophic gastritis (AG) in the antrum, corpus, or pangastritis: AGA, AGC, AGpan, respectively. In the proposed algorithm, the subsequent conduct should be clearly different in these three risk groups.

Starting from the group where GastroPanel^{®} test results in a normal marker profile, the continuation should depend on the symptoms. Patients with a normal marker profile and no longer reporting clinical symptoms, the likelihood is very low for a significant gastric pathology even in a long term, as shown by the longitudinal cohort studies [49]. Thus, these subjects can be safely returned to a regular path, with no need to additional examinations.

For symptomatic patients, with a normal GastroPanel^{®} marker profile, it is highly unlikely that a significant gastric pathology will be find on gastroscopy [42, 43, 46-48, 51]. Because of this, the next step should be the search of the origin of the symptoms from other sources. Of the Biohit tests [42], the logical next step is to institute ColonView^{®} Quick Test to exclude FOB [37]. Confirmation of a positive ColonView^{®} test necessitates the use of colonoscopy [40].

GastroPanel^{®} is the most comprehensive test for detection of Hp-infection, devoid of the caveats (false positive- and false negative results) inherent to the commonly used HP tests (i.e. 13C-UBT and stool antigen test) [46]. In this algorithm, GastroPanel^{®} detection of Hp-infection (with no AG) should prompt the eradication therapy, as instructed by the international consensus reports [7]. The success of this eradication should always be controlled. For this control, there are several options also among the Biohit tests: GastroPanel^{®}, HP Quick Test^{®} and the HP UFT300 Quick Test^{®} [42].

All patients having symptoms after Hp-eradication must be referred for gastroscopy. This enables direct visualization of Hp in the biopsies, and testing of the biopsy using either of the HP quick tests.

When GastroPanel^{®} biomarker profile indicates AG [40-51], the conduct should be straight forward. Gastroscopy with directed biopsies is mandatory to establish the severity of AG as well as its topography (AGA, AGC, AGpan) and possible accompanying IM or IN in the gastric mucosa. The authors are practically unanimous in that gastroscopy and directed biopsies are the gold standard detection for AG, because of the associated high risk for subsequent GC [7-21].

According to one embodiment, the method for non-invasive screening of a symptomless subject or subject having reflux, dyspeptic or other abdominal symptoms comprises at least the steps of:
- quantitatively measuring by an analytical immunoassay method concentration(s) of a) pepsinogen I (PGI), pepsinogen II (PGII), gastrin-17 (G-17) and *Helicobacter pylori* antibody (HpAb) biomarkers, or b) pepsinogen I (PGI) biomarker, or c) PGI biomarker, PGII biomarker and PGI/PGII biomarker ratio, or d) G-17 biomarker, or e) pepsinogen I (PGI), pepsinogen II (PGII), PGI/PGII biomarker ratio, gastrin-17 (G-17) and *Helicobacter pylori* antibody (HpAb) biomarkers from a blood, serum or plasma sample obtained from said subject,
- quantitatively measuring by an analytical immunoassay method concentration(s) of hemoglobin (Hb) and/or hemoglobin/haptoglobin complex (Hb/Hp) from a stool sample obtained from said subject,
- comparing the obtained value(s) to a pre-determined reference range(s) and/or cut-off value(s), and
- based on said comparison, detecting whether the subject is indicative for normal gastric function, *Helicobacter*-infection, atrophic gastritis and/or fecal occult blood.

According to a further embodiment, the method comprises running at least one, such as four, analytical immunoassay(s) comprising the PGI, PGII, G-17 and HpAb biomarkers at the same time from the same sample in one microplate and measuring said biomarker concentrations during 3 hours.

According to an even further embodiment, the PGI, PGII, G-17 and HpAb biomarker concentration(s), and the Hb and Hb/Hp biomarker concentration(s) measurements are prescribed simultaneously.

According to one embodiment, the biomarker value(s) is/are indicative for atrophic gastritis, and therefore also high risk of gastric cancer, if the PGI concentration in said sample is close to the lower limit or below the reference range or cut-off value, PGI/PGII ratio is close to the lower limit or below the reference range or cut-off value and G-17 concentration is close to the upper limit or above the reference range.

In the present context, the reference ranges for PGI value is 30 - 160 µg/l, for PGII 3-20 µg/l, for PGI/PGII ratio 3 or below 3, for G-17S (stimulated) value 5-30 pmol/l, for G-17B (fast) 2 - 10 pmol/l and for HpAb 0-30 EIU.

In the present context, the typical cut-off values for the biomarkers are selected from the group comprising: PGI 30 µg/l, PGI/PGII ratio 3, G-17S value 5 pmol/l, G-17B 2 pmol/l and HpAb 30 EIU.

Whenever GastroPanel^{®} test (ELISA or Quick test) shows a normal biomarker profile, the high NPV of the test confirms with high certainty that the symptoms are of extra-gastric origin. The logical next step is to exclude colorectal neoplasia as a potential cause, by testing for FOB with the ColonView^{®} test. Pending on the setting, the end user can select between ColonView^{®} Quick test or ColonView^{®} ELISA versions.

BIOHIT ColonView^{®} Quick test is a visual and automatic test, using immunochromatography for quick and qualitative detection of human hemoglobin (Hb) and hemoglobin/haptoglobin complex (Hb/Hp) in stool samples. Hb/Hp complex plays an important role in the retrieval of Hb from lysed erythrocytes and is quite resistant to acid and proteolytic degradation. This means that the Hb/Hp complex can be detected even after longer passage through the bowel, increasing the chance that also the blood mixed with larger intestinal polyps and proximal CRCs can be detected. There is evidence that detection of the Hb/Hp complex displays a significantly increased sensitivity in recognition of colorectal adenomas and carcinomas, when combined with Hb detection [52-56]. The results of ColonView^{®} Quick test are interpreted in two modes: 1) visual reading (VA) and 2) automatic reading (AA). For the automatic reading, the Quick Test Reader (QTR) is needed for quantitative evaluation of lateral flow assays (9-11). The device has been validated and configured by Biohit Oyj for the use in the AA reading mode of ColonView^{®} Quick test [54, 55].

The ColonView^{®} ELISA is intended for easy and automated detection of FOB in stool samples. The principal use of the Biohit ColonView^{®} ELISA test is to screen for lower intestinal pathologies, such as CRC and their precursors. However, with the combination of two biomarkers, the ColonView^{®} ELISA is sensitive enough to detect bleeding derived from both the lower and the upper gastrointestinal tracts, thanks to the excellent stability of the Hb/Hp complex [40-56].

The test is based on highly sensitive immunochemical detection of Hb and the Hb/Hp complex. The test utilizes a sandwich immunoassay (ELISA) with monoclonal antibodies specific to Hb or the Hb/Hp complex, respectively, adsorbed on a microplate and detection antibodies labeled with horseradish peroxidase (HRP). The test includes two microtiter plates (96 wells): the first plate is coated with Hb-specific antibody, and the second plate with an antibody specific to Hp [54, 55]. A stool sample is collected by the patient (using the provided Stool Collection Paper and the sampling device) into the sample tube containing sample buffer. In the sample buffer, the specimen(s) can be stored in the refrigerator (2-8 °C) for no longer than 11 days, or at room temperature (max. 25°C) for no longer than 5 days.

Optimal sensitivity in FOB detection is achieved by testing three consecutive fecal samples for both Hb and Hb/Hp complex (ColonView^{®}), far superior to the sensitivity achieved by i) the FITs using Hb testing only, or by ii) any test based on analysis of one fecal sample only [57-62].

Dyspepsia belongs among the most frequent clinical symptoms complained by the patients within the primary health care. The diversity of clinical conditions behind the symptoms are far too non-specific to preclude the correct diagnosis on clinical grounds alone, and emphasizes the need for a cogent algorithms [23-25]. Such an algorithm is proposed here, based on a targeted use of two extensively validated non-invasive tests: GastroPanel^{®} and ColonView^{®}.

According to one embodiment, the method further comprises at least the steps of:
- collecting stool of a subject during one or more days,
- mixing the samples from the stool of each collection day and dissolving it into a single unit of preservation liquid,
- shaking the sample in the preservation liquid and allowing the hemoglobin/haptoglobin complex to dissolve into the preservation liquid, whereby an extract is obtained, and
- carrying out said measurement on the obtained extract by using an immunoassay method.

In the present context, it is preferred that the stool is collected during two or more days, preferably three or four days.

According to a further embodiment, the Hb and/or Hb/Hp measurement is carried out on the obtained extract by using one single immunochemical test determining the amount of hemoglobin/haptoglobin complex in the extract.

According to even further embodiment, the hemoglobin/haptoglobin complex is dissolved after each sampling by shaking the sample and the preservation liquid.

In the present context, the analytical sensitivity for the Hb/Hp complex is 4 ng/ml.

According to one embodiment, the measurements are carried out by using an automated analytical immunoassay method, selected from the group of enzyme immunoassay (EIA), such as enzyme-linked immunosorbent assay (ELISA), radioimmunoassay (RIA), fluorescence immunoassay (FIA), or chemiluminescence immunoassay (CLIA), preferably ELISA.

Using GastroPanel^{®} test as the first-line test, it is possible to stratify the patients into three categories at different risk for GC. Because of the very high NPV of the GastroPanel^{®} test, the patients with normal biomarker profile can be safely examined for the extra-gastric origin of their symptoms. For these patients, ColonView^{®} is the rational first step to exclude or confirm the presence of occult blood (FOB) in the stool samples. All patients testing positive need colonoscopy to diagnose the origin and cause of FOB. All patients testing Hp-positive in GastroPanel^{®} should be treated for Hp, followed by appropriate control. All patients with AG need a referral for gastroscopy to confirm the grade and topography of AG and to exclude coexisting GC.

Replacing gastroscopy by GastroPanel^{®} as the first-line test in patients with dyspeptic symptoms, followed by ColonView^{®} test for all those testing GastroPanel^{®} negative can preserve invasive endoscopies (gastroscopy, colonoscopy) only for those patients testing positive with the respective tests. This leads to substantial savings in health care costs as compared with the usual practice of using endoscopy as the first-line diagnostic tool. It has been estimated that up to 80% of unnecessary gastroscopies and majority of colonoscopies could be avoided by screening the dyspeptic patients by GastroPanel^{®} and ColonView^{®} tests.

Thus, the use of the herein described method in detecting subjects having risk for contracting gastric cancer and/or lower gastrointestinal pathologies, such as colorectal cancer belongs to the scope of the present invention.

Current Care Guidelines [63] suggest that FOB testing in subjects having dyspeptic symptoms is not reasonable, but instead recommends performing endoscopic examination, which include invasive measures. In addition, the guidelines do not recommend pepsinogen or gastrin biomarker measurements for the detection of dyspepsia

(see also references [64-66]). These guidelines therefore lead a skilled person of the art away from the teachings of the present invention, which focuses merely on combination of non-invasive tests that provide quick and reliable results in an early stage of dyspepsia and other relating abdominal malfunctions and diseases.

According to the cost model designed by the Nordic Health Care Group (NHG), screening of the different risk factors of gastric-and esophageal cancer by GastroPanel^{®} test would save the life-time health care costs of 10 age groups (e.g. 65-74-year-olds) in Finland by over 800 million euros [67].

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

### EXAMPLE

According to one embodiment of the present invention, it is advantageous to measure both Hb and Hb/Hp-complex in stool, and not just Hp, which is not sufficient for the early detection of as many intestinal cancer adenoma precursors as possible. Table 1 below shows a comparison between one sample Hb-testing and one, two or three sample Hb- and Hb/Hp-testing (ColonView-FIT) in the diagnostics of gastric cancer and its precursors.

**Table 1**

| **EVENT** | **Sensitivity (95%CI)** | **Accuracy (95%CI)** | **PPV (95%CI)** | **NPV(95%CI)** | **AUC (95%CI)** |
|---|---|---|---|---|---|
| **ADENOMA** | | | | | |
| Hb-test 1 sample^{∗} | 45.1 (34.6 - 55.8) | 93.9 (87.8 - 97.5) | 85.4 (72.2 - 93.9) | 68.2 (60.3 - 75.4) | 0.690 (0.640 - 0.675) |
| Hb and Hb/Hp test 1 sample | 76.9 (66.9 - 85.1) | 88.6 (81.3 - 93.8) | 84.3 (74.7 - 91.4) | 82.8 (74.9 - 89.0) | 0.828 (0.775 - 0.880) |
| Hb and Hb/Hp test 2 samples^{∗∗} | 94.5 (87.6 - 98.2) | 85.1 (77.2 - 91.1) | 83.5 (74.9 - 90.1) | 95.1 (88.9 - 98.4) | 0.898 (0.858 - 0.938) |
| Hb and Hb/Hp test 3 samples^{∗∗∗} | 94.5 (87.6 - 98.2) | 85.1 (77.2 - 91.1) | 83.5 (74.9 - 90.1) | 95.1 (88.9 - 98.4) | 0.898 (0.858 - 0.938) |

| **ADENOMA** + **CARCINOMA** | | | | | |
|---|---|---|---|---|---|
| Hb-test 1 sample^{∗} | 70.3 (63.1 - 76.8) | 93.9 (87.8 - 97.5) | 94.9 (87.8 - 97.9) | 66.0 (58.2 - 73.3) | 0.820 (0.780 - 0.860) |
| Hb ja Hb/Hp test 1 sample | 88.2 (82.6 - 92.4) | 88.6 (81.3 - 93.8) | 92.7 (87.8 - 96.0) | 82.1 (74.2 - 88.4) | 0.884 (0.846 - 0.921) |
| Hb and Hb/Hp test 2 samples^{∗∗} | 97.3 (93.8 - 99.1) | 85.1 (77.2 - 91.1) | 91.4 (86.6 - 94.9) | 95.1 (88.9 - 98.4) | 0.910 (0.880 - 0.950) |
| Hb and Hb/Hp test 3 samples^{∗∗∗} | 97.3 (93.8 - 99.1) | 85.1 (77.2 - 91.1) | 91.4 (86.6 - 94.9) | 95.1 (88.9 - 98.4) | 0.910 (0.880 - 0.950) |

| **CARCINOMA** | | | | | |
|---|---|---|---|---|---|
| Hb-test 1 sample^{∗} | 94.7 (88.0 - 98.3) | 93.9 (87.8 - 97.5) | 92.7 (85.6 - 97.0) | 95.5 (89.9 - 98.5) | 0.940 (0.910 - 0.970) |
| Hb and Hb/Hp test 1 sample | 98.9 (94.3 - 100) | 88.6 (81.3 - 93.8) | 87.9 (80.1 - 93.4) | 990 (94.7 - 100) | 0.938 (0.907 - 0.969) |
| Hb and Hb/Hp test 2 samples^{∗∗} | 100 (96.2 - 100) | 85.1 (77.2 - 91.1) | 84.8 (76.8 - 90.9) | 100 (96.3 - 100) | 0.930 (0.890 - 0.960) |
| Hb and Hb/Hp test 3 samples^{∗∗∗} | 100 (96.2 - 100) | 85.1 (77.2 - 91.1) | 84.8 (76.8 - 90.9) | 100 (96.3 - 100) | 0.930 (0.890 - 0.960) |

| | | | | | |
|---|---|---|---|---|---|
| ^{∗}Hb-determination made from the only sample of the subject; ^{∗∗}Testing of two patient samples for Hb and Hb/Hp-complex: either of the two samples positive for Hb or Hb/Hp-complex; ^{∗∗∗}Testing of three patient samples for Hb and Hb/Hp-complex: any of the three samples positive for Hb or Hb/Hp-complex. | | | | | |

### CITATION LIST

1.Talley NJ, Zinsmeister AR, Schleck CD, Melton III LJ. Dyspepsia and dyspepsia subgroups: a population-based study. Gastroenterol 1992;102:1259-1268.
2.Drossman DA, Li Z, Andruzzi E, Temple RD, Talley NJ, Thompson WG. U.S. householder survey of functional gastrointestinal disorders. Prevalence, sociodemography and health impact. Dig Dis Sci 1993;38: 1569-1580.
3.Hansen JM, Bytzer P, Schaffalitzky D, Muckadell OB. Management of dyspeptic patients in primary care. Value of the unaided clinical diagnosis and of dyspepsia subgrouping. Scand J Gastroenterol 1998;33:799-805.
4.Heikkinen M, Pikkarainen P, Takala J, Julkunen R. General practitioners' approach to dyspepsia. Survey of consultation frequencies, treatment, and investigations. Scand J Gastroenterol 1996;31:648-653.
5.Majumdar SR, Soumerai SB, Farraye FA, Lee M, Kemp JA, Henning JM. Chronic acid-related disorders are common and under investigated. Am J Gastroenterol 2003;98:2409-2414.
6.Nebel OT, Fornes MF, Castell DO. Symptomatic gastroesophageal reflux: incidence and precipitating factors. Am J Digest Dis 1976;21:953-956.
7.Malfertheiner P, Megraud F, O'Morain CA, Atherton J, Axon AT, Bazzoli F, Gensini GF. Gisbert JP, Graham DY, Rokkas T, El-Omar EM, Kuipers EJ. European Helicobacter Study Group. Management of Helicobacter pylori infection--the Maastricht IV/ Florence Consensus Report. Gut 2012;61:646-664.
8.Sipponen P, Kekki M, Haapakoski J, Ihamäki T, Siurala M. Gastric cancer risk in chronic atrophic gastritis: statistical calculations of cross-sectional data. Int J Cancer 1985;35:173-177.
9.Israel DA, Peek RM. Review article: pathogenesis of Helicobacter pylori-induced gastric inflammation. Aliment Pharmacol Ther 2001;15:1271-1290.
10.Parsonnet J, Hansen S, Rodriguez L, Gelb AB, Wamke RA, Jellum E. Helicobacter pylori infection and gastric lymphoma. N Engl J Med 1994;330:1267-1271.
11.Khulusi S, Mendall MA, Patel P, Levy J, Badve S, Northfield TC. Helicobacter pylori infection density and gastric inflammation in duodenal ulcer and non-ulcer subjects. Gut 1995;37:319-324.
12.International Agency for Research on Cancer, World Health Organization. Schistosomiasis, liver flukes and Helicobacter pylori. IARC working group on the evaluation of carcinogenic risks to human. Monogr Eval Carcinog Risks Hum 1994;61:218-220.
13.Uemura N, Okamoto S, Yamamoto S, Matsumura N, Yamaguchi S, Yamakido M. Helicobacter pylori infection and the development of gastric cancer. N Engl J Med 2001;345:829-832.
14.Valle J, Kekki M, Sipponen P, Ihamäki T, Siurala M. Long-term course and consequences of Helicobacter pylori gastritis. Results of a 32-year follow-up study. Scand J Gastroenterol 1996;31:546-550.
15.Kuipers EJ, Uyterlinde AM, Peña AS, Roosendaal R, Pals G, Nelis GF. Long-term sequelae of Helicobacter pylori gastritis. Lancet 1995;345:1525-1528.
16.Correa P, Haenszel W, Cuello C, Zavala D, Fontham E and Zarama G: Gastric precancerous process in a high risk population: cohort follow-up. Cancer Res 1990;50:4737-4740.
17.Filipe MI, Munoz N, Matko I, Kato I, Pompe-Kim V, Jutersek A. Intestinal metaplasia types and the risk of gastric cancer: a cohort study in Slovenia. Int J Cancer 1994;57:324-329.
18.Ohata H, Kitauchi S, Yoshimura N, Mugitani K, Iwane M, Nakamura H. Progression of chronic atrophic gastritis associated with Helicobacter pylori infection increases risk of gastric cancer. Int J Cancer 2004;109: 138-143.
19.Correa P. A human model of gastric carcinogenesis. Cancer Res 1988;48:3554-3560.
20.Correa P. The epidemiology of gastric cancer. World J Surg 1991;15:228-234.
21.Correa P. Human gastric carcinogenesis: a multistep and multifactorial process - First American Cancer Society Award Lecture on Cancer Epidemiology and Prevention. Cancer Res 1992;52:6735-6740.
22.Weck MN, Stegmaier C, Rothenbacher D, Brenner H. Epidemiology of chronic atrophic gastritis: population-based study among 9444 older adults from Germany. Aliment Pharmacol Ther 2007;26:879-887.
23.Lomba-Viana R, Dinis-Ribeiro M, Fonseca F, Vieira AS, Bento MJ, Lomba-Viana H. Serum pepsinogen test for early detection of gastric cancer in a European country. Eur J Gastroenterol Hepatol 2012;24:37-41.
24.Bornschein J, Selgrad M, Wex T, Kuester D, Malfertheiner P. Serological assessment of gastric mucosal atrophy in gastric cancer. BMC Gastroenterol 2012;12:10. doi: 10.1186/1471-230X-12-10.
25.Germaná B, Di Mario F, Cavallaro LG, Moussa AM, Lecis P, Liatoupolou S, Comparato G, Carloni C, Bertiato G, Battiestel M, Papa N, Aragona G, Cavestro GM, Iori V, Merli R, Bertolini S, Caruana P, Franzé A. Clinical usefulness of serum pepsinogens I and II, gastrin-17 and anti-Helicobacterpylori antibodies in the management of dyspeptic patients in primary care. Dig Liver Dis 2005;37:501-508.
26.Denham JM, Hill ID. Celiac disease and autoimmunity: Review and controversies. Curr Allergy Asthma Rep 2013;13:347-353.
27.Catassi C, Anderson RP, Hill ID, Koletzko S, Lionetti E, Mouane N, Schumann M, Yachha SK. World perspective on celiac disease. JPGN 2012;55:494-499.
28.Di Sabatino A, Corazza GR. Coeliac disease. Lancet 2009;373:1480-1493.
29.Dahlqvist A, Hammond JB, Crane RK et al. Intestinal lactase deficiency and lactose intolerance in adults: preliminary report. Gastroenterol 1963; 45: 488-491.
30.Vesa TH, Marteau P, Korpela R. Lactose intolerance. J Am Coll Nutr 2000;19:165S-175S.
31.Joachim G. The relationship between habits of food consumption and reported reactions to food in people with inflammatory bowel disease--testing the limits. Nutr Health 1999;13:69-83.
32.Kuokkanen M, Myllyniemi M, Vauhkonen M, Helske T, Kääriäinen I, Karesvuori S, Linnala A, Härkönen M, Järvelä I, Sipponen P. A biopsy-based quick test in the diagnosis of duodenal hypolactasia in upper gastrointestinal endoscopy. Endoscopy 2006;38:708-712.
33.Tibble J, Teahon K, Thjodleifsson B, Roseth A, Sigthorsson G, Bridger S, Foster R, Sherwood R, Fagerhol M, Bjamason I. A simple method for assessing intestinal inflammation in Crohn's disease. Gut 2000;47:506-513.
34.Tibble J, Sighthorsson G, Foster R, Forgacs I, Bjarnason I. Use of surrogate markers of inflammation and Rome criteria to distinguish organic from nonorganic intestinal disease. Gastroenterol 2002:123:450-460.
35.Drossman DA, Thompson WG. The irritable bowel syndrome: review and a graduated multicomponent treatment approach. Ann Inter Med 1992;116:1009-1016.
36.Drossman DA. The functional gastrointestinal disorders and the Rome II process. Gut 1999;45:111-115.
37.Vasilyev S, Smimova E, Popov D, Semenov A, Eklund C, Hendolin P, Paloheimo L, Syrjänen K. A New-generation fecal immunochemical test (FIT) is superior to guaiac-based test in detecting colorectal neoplasia among colonoscopy referral patients. Anticancer Res 2015;35:2873-2880.
38.von Karsa L, Patnick J, Segnan Net al European guidelines for quality assurance in colorectal cancer screening and diagnosis: Overview and introduction to the full Supplement publication. Endoscopy 2013;45:51-59.
39.Segnan N, Patnick J, von Karsa Let al. European guidelines for quality assurance in colorectal cancer screening and diagnosis - First edition., eds. Luxembourg: European Commission, Publications Office of the European Union; 2010.
40.Mecklin J, Syrjänen K, Eskelinen M. Colorectal cancer (CRC) screening with traditional and new-generation fecal immunochemical tests (FIT): A critical review of fecal occult blood tests (FOBTs). Anticancer Res 2020;40:575-581.
41.Suovaniemi O. GastroPanel-tutkimus osaksi dyspepsian hoitokäytäntöä Yleislääkäri 2007;4:104-106.
42. http://www.biohithealthcare.com/products/diagnostics-tests
43.Aine R, Kahar E, Aitokari K, Salminen J, Eklund C, Paloheimo L, Peetsalu A, Syrjänen K. Atrophic gastritis (AG) and its clinical sequels among elderly people in Finland and Estonia. A comparative study using GastroPanel and B12-vitamin testing of the residents in assisted-housing facilities. J Aging Res Clin Pract 2016;5:194-202.
44.Syrjänen K. A Panel of serum biomarkers (GastroPanel®) in non-invasive diagnosis of atrophic gastritis. Systematic review and meta-analysis. Anticancer Res 2016;36:5133-5144.
45.Syrjänen K. Role of serological biomarker testing (GastroPanel®) in diagnosis of symptomatic dyspepsia and in screening of the risks of stomach cancer. EC Gastroenterol Digest Syst 2017;1(6):209-222.
46.Syrjänen K. Serological biomarker panel (GastroPanel®): A test for non-invasive diagnosis of dyspeptic symptoms and for comprehensive detection of Helicobacter pylori infection. Biomark J 2017;3:1-10.
47.Benberin V, Bektayeva R, Karabayeva R, Lebedev A, Akemeyeva K, Paloheimo L, Syrjänen K. Prevalence of H.pylori infection and atrophic gastritis among ymptomatic and dyspeptic adults in Kazakhstan. A Hospital-Based screening with a panel of serum biomarkers. Anticancer Res 2013;33:4595-4602.
48.Agréus L, Kuipers EJ, Kupcinskas L, Malfertheiner P, Di Mario F, Leja M, Mahachai V, Yaron N, van Oijen M, Perez Perez G, Rugge M, Ronkainen J, Salaspuro M, Sipponen P, Sugano K, Sung J. Rationale in diagnosis and screening of atrophic gastritis with stomach-specific plasma biomarkers. Scand J Gastroenterol 2012;47:136-147.
49. Kurilovich SA, Belkovets AV, Reshetnikov OV, Openko TG, Malyutina SK, Ragino YI, Scherbakova LV, Leja M, Paloheimo L, Syrjänen K, Voevoda MI. Stomach-specific biomarkers (GastroPanel) can predict the development of gastric cancer in Caucasian population: A longitudinal nested case-control study in Siberia. Anticancer Res 2016;36:247-254.
50.Syrjänen KJ, Sipponen P, Härkönen M, Peetsalu A, Korpela S. Accuracy of GastroPanel testing in detection of atrophic gastritis. Eur J Gastroenterol Hepatol 2015;27:102-104.
51.Zagari RM, Rabitti S, Greenwood DC, Eusebi LH, Vestito A, Bazzoli F. Systematic review with meta-analysis: diagnostic performance of the combination of pepsinogen, gastrin-17 and anti-Helicobacter pylori antibodies serum assays for the diagnosis of atrophic gastritis. Aliment Pharmacol Ther 2017;1-11
52.Sieg A, Thoms C, Lüthgens K, John MR, Schmidt-Gayk H. Detection of colorectal neoplasms by the highly sensitive hemoglobin-haptoglobin complex in feces. Int J Colorectal Dis 1999;14:267-271.
53.Lüthgens K, Maier A, Kampert I, Sieg A, Schmidt-Gayk H. Hemoglobin-Haptoglobin complex: A highly sensitive assay for the detection of fecal occult blood. Clin Laborat 1998;44:543-551.
54. https://www.biohithealthcare.com/en/products/diagnostic-tests/colonview-quick-test/
55. https://www.colonview.fi/en/
56.Syrjänen K. Suomalainen FIT-testi: entistä tehokkaampaa suolistosyövän seulontaa. BestPractice Onkologia 2013;6:10-14.
57.Vasilyev S, Smimova E, Popov D, Semenov A, Eklund C, Hendolin P, Paloheimo L, Syrjänen K. A new-generation fecal immunochemical test (FIT) is superior to guaiac-based test in detecting colorectal neoplasia among colonoscopy referral patients. Anticancer Res 2015;35: 2873-2880.
58.Guimarães DP, Fregnani JH, Reis RM, Taveira LN, Scapulotempo-Neto C, Matsushita M, Silva SR, Oliveira CZ, Longatto-Filho A, Eklund C, Paloheimo LI, Mauad E, Syrjänen K. A new-generation fecal immunochemical test compared with guaiac fecal occult blood test for colorectal neoplasia detection in colonoscopy referral patients. Anticancer Res 2019;39:261-269.
59.Meklin J, Eskelinen M, Guimaraes DP, Selander T, Inkinen J, Tiusanen T, Syrjänen K, Eskelinen M. The new generation immunochemical test for fecal occult blood (Colon View quick test) shows a high diagnostic accuracy in colorectal cancer detection. Anticancer Res. 2021;41: 5071-5079.
60.Meklin J, Eskelinen M, Guimaraes DP, Selander T, Tiusanen T, Syrjänen K, Eskelinen M. The automatically analyzed (AA) Colon View (CV) Quick Test for fecal occult blood shows higher diagnostic accuracy in detection of colorectal adenoma than visually analyzed test. Anticancer Res 2021;41:5517-5525.
61.Lee JK, Liles EG, Bent S, Levin TR, Corley DA. Accuracy of fecal immunochemical tests for colorectal cancer: Systematic review and meta-analysis. Ann Intern Med 2014;160: 171-181.
62.Yamamoto M, Nakama H. Cost-effectiveness analysis of immunochemical occult blood screening for colorectal cancer among three fecal sampling method. Hepatogastroenterol 2000;47:396-399.
63. https://www.kaypahoito.fi/hoi50093
64. www.biohithealthcare.com/Critisism-about-current-care-recommendations
65. www.biohithealthcare.com/additional-information
66. Immunoassay for fecal human hemoglobin; https://patents.google.com/patent/US4427769
67. https://www.gastropanel.com/decision-makers/screening-model

## Claims

1. A method for non-invasive screening of a symptomless subject or subject having reflux, dyspeptic or other abdominal symptoms, **characterized in that** the method comprises at least the steps of:
- quantitatively measuring by an analytical immunoassay method concentration(s) of a) pepsinogen I (PGI), pepsinogen II (PGII), gastrin-17 (G-17) and *Helicobacter pylori* antibody (HpAb) biomarkers, or b) pepsinogen I (PGI) biomarker, or c) PGI biomarker, PGII biomarker and PGI/PGII biomarker ratio, or d) G-17 biomarker, or e) pepsinogen I (PGI), pepsinogen II (PGII), PGI/PGII biomarker ratio, gastrin-17 (G-17) and *Helicobacter pylori* antibody (HpAb) biomarkers from a blood, serum or plasma sample obtained from said subject,
- quantitatively measuring by an analytical immunoassay method concentration(s) of hemoglobin (Hb) and/or hemoglobin/haptoglobin-complex (Hb/Hp) from a stool sample obtained from said subject,
- comparing the obtained value(s) to a pre-determined reference range(s) and/or cut-off value(s), and
- based on said comparison, detecting whether the subject is indicative for normal gastric function, *Helicobacter*-infection, atrophic gastritis and/or fecal occult blood.

2. The method according to claim 1, **characterized in** running at least one, such as four, analytical immunoassay(s) comprising the PGI, PGII, G-17 and HpAb biomarkers at the same time from the same sample in one microplate and measuring said biomarker concentrations during 3 hours.

3. The method according to claim 1 or 2, **characterized in that** the PGI, PGII, G-17 and HpAb biomarker concentration(s), and the Hb and/or Hb/Hp biomarker concentration(s) measurements are prescribed simultaneously.

4. The method according to any of the preceding claims, **characterized in that** the biomarker value(s) is/are indicative for atrophic gastritis, and therefore also high risk of gastric cancer, if the PGI concentration in said sample is close to the lower limit or below the reference range or cut-off value, PGI/PGII ratio is close to the lower limit or below the reference range or cut-off value and G-17 concentration is close to the upper limit or above the reference range.

5. The method according to any of the preceding claims, **characterized in that** the reference ranges for PGI value is 30 - 160 µg/l, for PGII 3-20 µg/l, for PGI/PGII ratio 3 or below 3, for G-17S (stimulated) value 5-30 pmol/l, for G-17B (fast) 2 - 10 pmol/l and for HpAb 0 - 30 EIU.

6. The method according to any of the preceding claims, **characterized in that** the typical cut-off values for the biomarkers are selected from the group comprising: PGI 30 µg/l, PGI/PGII ratio 3, G-17S value 5 pmol/l, G-17B 2 pmol/l and HpAb 30 EIU.

7. The method according to any of the preceding claims, **characterized in that** the method further comprises at least the steps of:
- collecting stool of a subject during one or more days,
- mixing the samples from the stool of each collection day and dissolving it into a single unit of preservation liquid,
- shaking the sample in the preservation liquid and allowing the hemoglobin/haptoglobin complex to dissolve into the preservation liquid, whereby an extract is obtained, and
- carrying out said measurement on the obtained extract by using an immunoassay method.

8. The method according to any of the preceding claims, **characterized in** collecting stool samples of the subject during two or more days, preferably three or four days.

9. The method according to any of the preceding claims, **characterized in that** Hb and/or Hb/Hp measurement is carried out on the obtained extract by using one single immunochemical test determining the amount of hemoglobin/haptoglobin complex in the extract.

10. The method according to any of the preceding claims, **characterized in that** the analytical sensitivity for the Hb/Hp complex is 4 ng/ml.

11. The method according to any of the preceding claims, **characterized in that** the measurements are carried out by using an automated analytical immunoassay method, selected from the group of enzyme immunoassay (EIA), such as enzyme-linked immunosorbent assay (ELISA), radioimmunoassay (RIA), fluorescence immunoassay (FIA), or chemiluminescence immunoassay (CLIA), preferably ELISA.

12. Use of the method according to any of the preceding claims 1 to 11 in detecting subjects having risk for contracting gastric cancer and/or lower gastrointestinal pathologies, such as colorectal cancer.
